Europäisches Patentamt

European Patent Office

Office européen des brevets

(19)

(11) Veröffentlichungsnummer: **0 121 679**
**A1**

## (12) EUROPÄISCHE PATENTANMELDUNG

(21) Anmeldenummer: **84101298.2**

(22) Anmeldetag: **29.07.82**

(51) Int. Cl.³: **A 61 M 25/02,** A 61 F 13/00,
A 61 F 13/02

(30) Priorität: **29.07.81 DE 3129931**

(43) Veröffentlichungstag der Anmeldung: **17.10.84**
**Patentblatt 84/42**

(84) Benannte Vertragsstaaten: **AT BE CH FR GB IT LI LU NL SE**

(60) Veröffentlichungsnummer der früheren Anmeldung nach Art. 76 EPÜ: **0076896**

(71) Anmelder: **Institut für wissenschaftliche Photographie und Kinematographie Willi Fischer, Königin-Luise-Strasse 73, D-1000 Berlin 33 (DE)**

(72) Erfinder: **Fischer, Willi, Königin-Luise-Strasse 73, D-1000 Berlin 33 (DE)**

(74) Vertreter: **Von Samson-Himmelstjerna, Friedrich R., Dipl.-Phys. et al, Donaustrasse 24, D-8000 München 80 (DE)**

(54) **Medizinischer Verband zur Fixierung einer Sonde.**

(57) Medizinischer Verband zur Festlegung einer durch die Haut (20) eines Lebewesens in dessen Körper eingeführten Sonde (Nadel, Kanüle, Katheter oder dergleichen) (14) mit einem an der Haut (20) und am aus der Haut (20) herausragenden Teil der Sonde (14) gleichzeitig befestigbaren Verbandsabschnitt, der zur patientenschonenden guten Fixierung zur Anlage an der Haut (20) ausgelegtes Flächengebilde (1) und einen mit dem Flächengebilde (1) verbundenen und an diesem stutzenartig vorstehenden Fortsatz (4) mit einer daran zur Anlage an den aus der Haut (20) herausragenden Teil der Sonde (14) ausgeformten Anlagefläche (4'') nach Art einer oben offenen Rinne aufweist.

0121679

F. von SAMSON-HIMMELSTJERNA
PATENTANWALT
DIPL.-PHYS.
DONAUSTR. 24
D-8000 MÜNCHEN 80

# SAMSON

## PATENTANWALTSKANZLEI

TELEFON: 089/982277
TELEGRAMM: SAMPAT
TELEX: 524769 WAHBG
TELEKOPIERER: 089/984220
(GRUPPE 2 + 3)

Ausscheidung aus

Anmeldung Nr. 82 106 833.5

Anm.: Institut für wissenschaftliche Photographie und Kinematographie

U.Z.: 213/4-84 EP Pat

München, den

8. Februar 1984

vS/l/os

## Medizinischer Verband zur Fixierung einer Sonde

Die Erfindung bezieht sich auf einen medizinischen Verband zur Fixierung einer Sonde gemäß dem Oberbegriff des Anspruchs 1.

Der Ausdruck "Sonde" wird im Rahmen dieser Anmeldung als Sammelbegriff für Sonden im engeren Sinne, Nadeln, Kanülen, Katheter oder dgl. verwendet. Die Sonde kann hierbei zur Zuführung oder Abführung von Substanzen in oder aus dem Körper dienen; ebenso zur Ableitung von Informationen über Funktionsabläufe im Körperinneren des Lebewesens. Im lezteren Fall muß die Sonde nicht unbedingt hohl sein.

Aus der medizinischen Praxis ist es bereits bekannt, eine Sonde beispielsweise mittels eines Heftpflasterstreifens in Form eines sogenannten "V-Verbandes" auf der Haut zu fixieren. Bei dem bekannten "V-Verband" wird der Heftpflasterstreifen nach Art eines Schals einmal um den aus der Haut herausragenden Teil der Sonde geschlagen, wobei sich der Heftpflasterstreifen auf der der Haut gegenüberliegenden Seite der Sonde überkreuzt und von dort weitergeführt und an die Haut geklebt wird. Dieser bekannte "V-Verband" hat den Vorteil eines bequemen Anbringens. Auch ist er in der Regel medizinisch unbedenklich, insbesondere hautverträglich. Er hat jedoch den Nachteil, daß er nur eine vergleichsweise dürftige Fixierung bzw. Festlegung der Sonde erlaubt. Dies hat nicht nur die Gefahr eine Schmerzverursachung des Lebewesens bei Bewegung der Sonde zur Folge, sondern darüberhinaus auch die erheblich schwerwiegendere Gefahr einer unmittelbaren Verletzung, insbesondere einer Venenreizung oder noch schlimmer eines Venendurchstoßes und damit einhergehender erhöhter Gefahr einer Trombosebildung. Hinzu kommt, daß eine wiederholte Schmerzverursachung bei dem mit der Sonde behafteten Lebewesen zu Unruheerscheinungen und daraus folgend zu einer erhöhten Gefahr der Bewegung der Sonde führt. Schließlich besteht auch bei dem bekannten "V-Verband" die Gefahr, daß die Sonde abknickt, insbesondere nachdem die Mandrinnadel entfernt ist. Nicht nur die Bildung von Tromben, sondern darüberhinaus auch

0121679

ein Abknicken der Sonde kann zu verheerenden Schädigungen-bis hin zum Eintritt des Todes-des mit der Sonde behafteten Lebewesens führen. Gerade Katheter, die über die Vena subclavia und Vena jugularis interna eingeführt werden, sind dafür bekannt, daß sie sich leicht verschieben und gegebenenfalls knicken. Um ein Verschieben der Katheter zu vermeiden, näht man dieselben daher häufig durch eine einzelne starke Seitennaht an der Haut an. Diese Maßnahme stellt zwar eine sichere Fixierung der Sonde am Lebewesen dar. Sie ist aber wenig schonend. Ähnliche Probleme bestehen auch bei Kathetern, die in der Periduralanästhesie, sowie der Peridurographie zur Darstellung des Epiduralraumes durch Kontrastmittel verwendet werden.

Anstelle des oder zusätzlich zum bekannten ~~gattungsge-mäßen~~ "V-Verband" aus Heftpflastern wird bisweilen nach Einführung der Sonde durch den Arzt, insbesondere bei längerer Verweildauer ein Verband angelegt. Zwar findet hierdurch eine gewisse zusätzliche, dennoch aber unzureichende Fixierung der Sonde statt. Hinzu kommt, daß das Anlegen des Verbandes zeitraubend, häufig schmerzhaft, zumindest aber wegen der beim Wickeln des Verbandes wiederkehrenden Berührungen zwischen Verband und Sonde beunruhigend für den Patienten ist - mit der Folge, daß sich auch der Patient bewegt und eine schmerzhafte Berührung zwischen Verband und Sonde erst recht eintritt. Im übrigen weist dieser bekannte Verband im wesentlichen die gleichen Nachteile wie der bekannte ~~gattungsgemäße~~ "V-Verband" auf, wenngleich möglicherweise in abgeschwächter Form.

Institut für wissenschaftliche
Photographie und Kinematographie
Willi Fischer

Vorstehende Problematik wird bereits seit langem von den Fachleuten intensiv behandelt. So sind beispielsweise Versuche zur Lösung der oben genannten Probleme aus folgenden Druckschriften bekannt: US-PS 3 683 911; DE-OS 29 09 302; US-PS 3 834 380 und US-PS 3 568 679. Zwar können eine Reihe von Nachteilen der eingangs geschilderten, in der medizinischen Praxis verwendeten Verbände mit den aus den vorstehenden Druckschriften bekannten medizinischen Verbänden zur Fixierung einer Sonde behoben werden. Auch eignen sich die letztgenannten Verbände großenteils zur Herstellung als Massenartikel. Dennoch hat nach Kenntnis des Anmelders keiner der aus den vorstehenden Druckschriften bekannten Verbände Eingang in die medizinische Praxis zum Zwecke der Fixierung einer Sonde gefunden.

Der medizinische Verband zur Fixierung einer Sonde gemäß dem Oberbegriff des Anspruchs 1 ist beispielsweise aus der bereits genannten US-PS 3 683 911 bekannt. Dieser vorbekannte medizinische Verband hat den Vorteil einer ausgezeichneten Fixiermöglichkeit der Sonde an der Haut. Sind hierbei beispielsweise die Anlagefläche des Fortsatzes und die der Haut zugewandte Seite des Flächengebildes mit einer Haftschicht überzogen, so genügt ein leichtes Andrücken des Verbandsabschnittes einerseits auf die Haut und andererseits auf die Sonde, um zu einer vorgleichsweise raschen, für den Patienten schmerzlosen, gleichwohl aber sicheren Fixierung der Sonde zu gelangen. Einhergehend mit der erhöhten Fixierung der Sonde ergibt sich eine Verringerung der Infektionsgefahr, die beispielsweise durch Reizungen an der Vene und/oder durch Verschlechterung des Hautschlusses an der Punktionsstelle - infolge erhöhter Beweglichkeit - bedingt ist. Insbesondere werden aber bereits durch den bekannten Verband die infolge von Trombosen oder Unterbrechungen der Zu- oder Abfuhr von Substanzen in oder aus dem Körper bedingte lebensbedrohenden Gefahren beseitigt. Wegen der absoluten Fixierung der Sonde bietet bereits der bekannte Verband gute Anwendungsmöglichkeiten in der Periduralanästhesie sowie der Peridurographie zur Darstellung des Epiduralraumes. Bei dem aus der US-PS 3 683 911 bekannten medizinischen Verband ist der Fußbereich des Fortsatzes allseitig vom Flächengebilde umgeben, wobei das Flächengebilde in diesem Bereich eine Durchtrittsöffnung für die Sonde aufweist. Hierdurch wird nicht nur der Vorteil einer großflächigen Abdeckung des Applikationsortes und damit eine Verringerung der Infektionsgefahr erreicht. Hinzu kommt eine großflächige Ableitung von gegebenenfalls auf den Fortsatz oder die Sonde ausgeübten Druck- und/oder Zugkräften. Im Gegensatz hierzu werden bei dem aus der medizinischen Praxis bekannten "V-Verband" derartige Zug- und/oder Druckkräfte unmittelbar auf die Punktionsstelle und über die Sondenspitze zusätzlich noch auf die Gefäßwände im Innern des Blutgefäßes übertragen. Der vorbekannte medizinische Verband besteht aus Kunststoff, vorzugsweise Polyäthylen. Dies hat zwei Vorteile. Zum einen ist er in der Regel für sichtbares Licht, häufig auch für Röntgenstrahlen durchlässig. Hierdurch können Veränderungen, beispielsweise Entzündungen, Hautallergien oder dgl. im Bereich der Punktionsstelle frühzeitig erkannt und - wegen der frühzeitigen Erkennung - mit vergleichsweise schonenden Mitteln behandelt werden. Die Röntgenstrahlen-Durchlässigkeit ermöglicht darüber hinaus eine gegebenenfalls erforderlich werdende bequeme transvenöse Extraktion von Tromben durch Schlingen. Als weiterer Vorteil kommt hinzu, daß das Flächengebilde wegen seiner Beschaffenheit aus Kunststoff bequem der Ausformung der Oberfläche des Patienten im Bereich der Punktionsstelle angepaßt werden kann. Eine Verringerung der Gefahr einer Kontamination durch pathogene Erreger und damit eine erhöhte Schonung des Lebewesens wird bei dem bekannten medizinischen Verband dadurch bewirkt, daß die Haftschicht auf der dem Fortsatz abgewandten Fläche des Flächengebildes mit einem antibakteriellen Mittel versehen ist. Ferner ist das Flächengebilde von einem Einschnitt bzw. einer Schnittlinie durchzogen, die am äußeren Rand der für die Sonde vorgesehenen Durchtrittsöffnung beginnt und im wesentlichen unterhalb, d.h. in Längsrichtung des Fortsatzes bis zur äußeren Umgrenzungskante des Flächengebildes führt. Durch Zug an den zu beiden Seiten des Einschnittes liegenden Abschnitten des Flächengebildes können der Einschnitt und die Durchtrittsöffnung erweitert werden. Dies hat beim Anlegen und Abnehmen des Verbandes den Vorteil, daß er bei eingeführter Sonde bequem unter die Sonde geschoben werden kann. Auch diese Maßnahme dient demnach der Schonung des Patienten. Zur Erhöhung der Sterilität des Applikationsortes weist der bekannte Schnellverband eine von der freien Innenkante des Einschnittes ausgehende, den gesamten Einschnitt überlappende Lasche auf, die vorzugsweise mit einer selbstklebenden Haftschicht bestückt ist. Bei dem bekannten medizinischen Verband sind der Fortsatz und das Flächengebilde einstückig, vorzugsweise aus demselben Material hergestellt. Diese Maßnahme dient in besonderer Weise auch dazu, den Verband als Massenartikel herstellen zu können.

Bei dem aus der US-PS 3 683 911 bekannten medizinischen Verband ist der Fortsatz in Form eines geschlitzten Rohres ausgebildet. Die längs der Schlitzlinie aneinandergrenzenden freien Kanten des Fortsatzrohres sind voneinander wegschwenkbar - und wieder zusammenführbar -, da der Fortsatz ebenfalls aus einem elastischen Kunststoff hergestellt ist. Anders ausgedrückt, kann der Fortsatz auch als ein aus zwei im wesentlichen halbzylindrischen Rinnen aufgebautes Rohr angesehen werden. Um nach Einführung der Sonde den medizinischen Verband möglichst bequem in Wirkstellung bringen zu können, stoßen die beiden durch den rohrförmigen Fortsatz einerseits und das Flächengebilde andererseits geführten Schlitze an ein und demselben Punkt des äußeren Randes der Durchtrittsöffnung aneinander. Diese Maßnahme soll in besonderem Maße dem bequemen Öffnen bzw. Erweitern der Schlitze und der Durchtrittsöffnung dienen. Zwar mag es noch relativ einfach sein, den vorbekannten medizinischen Verband nach Einführung der Sonde in Wirkstellung zu bringen. Ist aber der Verband auf der Haut des Lebewesens und an der Sonde erst einmal befestigt, insbesondere mittels der Haftschicht verklebt, dann ist ein Entfernen des Verbandes bzw. ein Auswechseln desselben ein vergleichsweise aufwendiger und - wegen der hierbei erforderlichen Reiß- und Zerrbewegungen - ein für den Patienten schmerzhafter Vorgang, der insbesondere die Gefäße des Patienten reizen und gegebenenfalls ein Abknicken der Sonde zur Folge haben kann.

Auch aus der DE-OS 29 01 302 ist ein medizinischer Verband zur Festlegung einer Sonde bekannt, der im wesentlichen die Merkmale des medizinischen Verbandes gemäß dem Oberbegriff des Anspruchs 1 aufweist. Auch dieser Verband soll der Lösung der eingangs genannten Probleme dienen. Er hat im wesentlichen die gleichen Nachteile wie der aus der US-PS 3 683 911 bekannte Verband. Im übrigen gibt er zu dem Problem einer einfachen Applizierung bzw. Auswechselung des Verbandes zwei, gegebenenfalls miteinander kombinierbare Lösungen an. Einerseits nämlich wird eine um die Sonde innerhalb des Fortsatzes angeordnete Metalltülle vorgesehen. Die Metalltülle soll dazu dienen, ein Durchschneiden der Sonde für den Fall zu verhindern, daß der Fortsatz durch Aufschneiden von der Sonde gelöst werden soll. Andererseits sind zwei Sollbruchstellen bzw. Abreißstellen innerhalb des Fortsatzes vorgesehen. Diese Abreißstellen dienen dazu, den Fortsatz an wohldefinierten Stellen zur Ablösung von der Sonde abreißen zu können. Demnach sind zu dessen Entfernung bzw. Auswechslung nicht nur die bereits genannten Zerr- und Reißbewegungen, sondern auch dessen Zerstörung erforderlich.

Aus der DE-AS 15 66 588 ist ein Kathetereinführer - nicht ein medizinischer Verband zur Fixierung einer Sonde - bekannt, der als axial bewegliche Klemmhülse ausgestaltet ist. Auch er besteht im wesentlichen aus einem geschlitzten Rohr bzw. aus zwei halbzylindrischen, einander gegenüberliegenden Rinnen. In Wirkstellung und in Ruhestellung umschließt die Klemmhülse die Sonde im wesentlichen vollständig.

Ausgehend von vorgenanntem Stand der Technik liegt der Erfindung die Aufgabe zugrunde, den im Oberbegriff des Anspruchs 1 angegebenen Verband zur Fixierung einer Sonde derart weiterzuentwickeln, daß er unter möglichst weitgehender Beibehaltung seiner bisherigen Vorteile für das mit der Sonde behaftete Lebewesen schonender ist, insbesondere schnell, einfach und sicher anlegbar oder auswechselbar ist und sich zur Herstellung als Massenartikel eignet.

Diese Aufgabe wird dadurch gelöst, daß der Fortsatz des medizinischen Verbandes zur Fixierung einer Sonde nach dem Oberbegriff des Anspruchs 1 nach Art einer oben offenen, den aus der Haut herausragenden Teil der Sonde abstützenden Rinne ausgebildet ist.

Die Erfindung wendet sich demnach von den vorbekannten Lehren, die Sonde mittels des Fortsatzes im wesentlichen vollständig zu umschließen, ab. Vielmehr übernimmt der Fortsatz wegen seiner genannten Ausgestaltung im wesentlichen zwei Funktionen, nämlich einerseits die bereits angesprochene Abstützfunktion, damit sich die Sonde, insbesondere eine Nadel, nicht um ihren Drehpunkt, d.h. die Einstichstelle, in Richtung der Haut bewegen und damit die Vene durchstechen kann. Andererseits wird eine seitliche Verschiebung quer zur Sondenachse verhindert. Demgemäß ist die Rinne (maximal) halbzylindrisch ausgebildet. Durch diese Maßnahmen erzielt der erfindungsgemäße medizinische Verband im wesentlichen sämtliche Vorteile der vorbekannten Lösungen. Er gewährleistet darüber hinaus aber auch ein schnelleres, bequemeres und für den Patienten besonders schmerzfreies Verbinden oder Lösen des Verbandes mit bzw. von der Sonde. Denn hierzu muß der Verband lediglich unter die Sonde geschoben bzw. unter der Sonde weggezogen werden. Danach bzw. davor kann er beispielsweise bequem mit zwei einfachen Heftpflasterstreifen an der Rinne fixiert werden. Offensichtlich bedarf es zur Trennung des Verbandes von der Sonde oder zum Auswechseln desselben weder der obengenannten Zerr- und Reißbewegungen noch seiner Zerstörung. Hinzu kommt noch, daß auch die Herstellungskosten des Verbandes erheblich reduziert werden können. Denn pro Fortsatz wird allenfalls nur halb so viel Material benötigt wie für die Fortsätze in den vorbekannten medizinischen Verbänden. Auch die Anpassung einer rinnenförmigen, im wesentlichen also halbzylindrischen Fläche an eine Sonde ist erheblich einfacher als die Anpassung einer vollzylindrischen bzw. hohlzylindrischen Fläche. Auch bedarf es nicht des Anbringens eines Schlitzes im Hohlzylinder. Nach alledem löst die Erfindung das eingangs genannte, intensiv behandelte Problem mit besonders einfachen Mitteln, die zudem noch eine Reihe von Vorteilen für den Patienten und die Herstellung des Verbandes nach sich ziehen.

Gemäß einer bevorzugten Ausführungsform des medizinischen Verbandes ist der Fortsatz über eine Verbindungsrippe mit dem Flächengebilde verbunden. Hierdurch wird die Abstützfunktion der Rinne sowie die Übertragbarkeit von auf den Fortsatz wirkenden Kräften auf das gesamte Flächengebilde erhöht (Anspruch 2).

Statt der Verwendung von Heftpflasterstreifen zur weiteren Fixierung der Sonde an der Rinne weist vorzugsweise die Anlagefläche selbst eine Haftschicht auf. Hierdurch kann die Sonde durch leichtes Andrücken gegen den Fortsatz bequem fixiert werden (Anspruch 3).

Gemäß einer weiteren bevorzugten Ausführungsform ist der Fußbereich des Fortsatzes allseitig vom Flächengebilde umgeben, wobei das Flächengebilde in diesem Bereich eine Durchtrittsöffnung aufweist (Anspruch 4). Auch sind vorzugsweise der Fortsatz und das Flächengebilde aus demselben Material hergestellt (Anspruch 5). Die beiden vorstehend genannten Maßnahmen haben für sich gesehen im wesentlichen die gleichen Vorteile wie die entsprechenden, aus der US-PS 3 683 911 bekannten Maßnahmen (nämlich großflächige Ableitung von auf den Fortsatz ausgeübten Druck- und/oder Zugkräften und wirtschaftliche Herstellbarkeit).

Der erfindungsgemäße Verband eignet sich in besonderem Maße für die Fixierung von Sonden, die für Infusionen bzw. intravenöse Medikamenten-Applikationen oder die parenterale Ernährung vorgesehen sind. Ist die Anlagefläche auch zumindest teilweise einem an der Sonde vorhandenen Abschlußkonus angepaßt, so wird gerade die am Übergang des Anschlußkonus zur Sonde bestehende Knickgefahr verringert. Auch eignet sich der erfindungsgemäße Verband in erhöhtem Maße für Punktionskatheter, da er durch seine sterile und antibiotische Abdeckung erheblich die Infektzahl, speziell auch bei der Katheterisierung der Venae sectio, verringert.

Nachstehend wird die Erfindung anhand von Ausführungsbeispielen unter Bezugnahme auf die beigefügten schematischen Zeichnungen noch näher erläutert.

In den Zeichnungen zeigen:

Figur 1       ein erstes Ausführungsbeispiel in Wirkstellung; und

Figur 2       eine Ansicht eines weiteren Ausführungsbeispiels von unten.

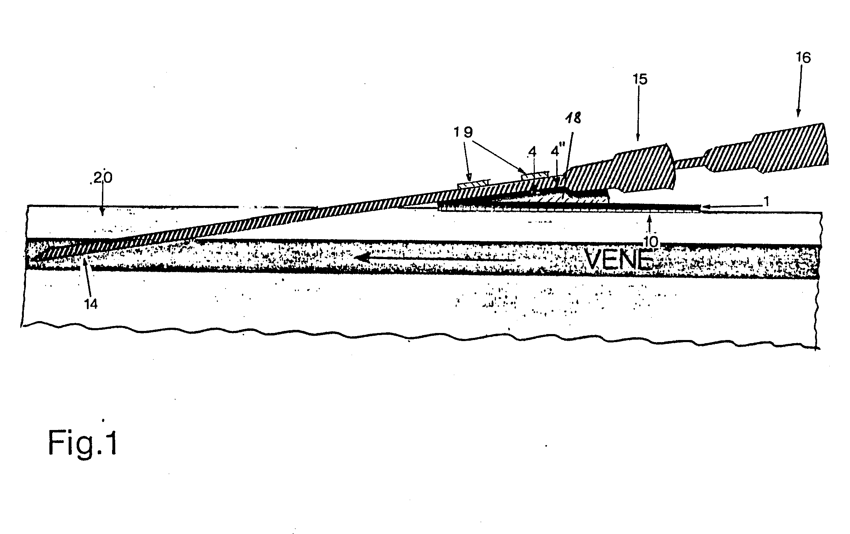

Figur 1 zeigt eine erstes Ausführungsbeispiel eines auf der Haut 20 eines Lebewesens befestigten erfindungsgemäßen medizinischen Verbandes. Der Verband dient zur Festlegung einer in die Vene des Lebewesens eingeführten Sonde 14. Hierzu weist der Verband im dargestellten Ausführungsbeispiel ein als biegsames Plättchen ausgebildetes Flächengebilde 1 auf, das auf seiner Unterseite mit einer auf der Haut 20 haftenden Haftschicht 10 beschichtet ist. Die Eigensteifigkeit und die Stärke des Plättchens sind so bemessen, daß es bequem Körperrundungen anpaßbar ist. Die Größe des Plättchens ist

0121679

so bemessen, daß es mittels der Haftschicht 10 einen ausreichenden Halt auf der Haut 20 findet. Von einer freien Kante des Plättchens ausgehend erstreckt sich ein Fortsatz 4 in spitzem Winkel zum Plättchen nach oben. Der Fortsatz ist hierbei als Rinne ausgebildet, deren Innenfläche der Außenfläche de Sonde 14, einschließlich dessen Anschlußkonus 15 angepaßt ist. Die Innenfläche des rinnenartigen Fortsatzes 4 dient demnach als Anlagefläche 4" für den aus der Haut 20 herausragenden Teil der Sonde 14. Der Winkel zwischen dem Plättchen und der Anlagefläche 4" des rinnenartigen Fortsatzes 4 ist so bemessen, daß eine unter üblichem Winkel in die Vene eingeführte Sonde 14 satt auf der Anlagefläche 4 aufliegt. Der Fortsatz 4 und das Flächengebilde 1 bestehen im dargestellten Ausführungsbeispiel aus dem gleichen Material. Zur starren Fixierung des Fortsatzes 4 am Plättchen 1 ist im dargestellten Ausführungsbeispiel zusätzlich noch eine Verbindungsrippe 18 zwischen dem Plättchen und der Unterseite des Fortsatzes 4 vorgesehen. Das Plättchen, der Fortsatz 4 und die Verbindungsrippe 18 können beispielsweise aus einem einstückig gegossenen Kunststoff bestehen.

Infolge der Rinnenform und der Anpassung der Innenfläche der Rinne an die Außenfläche der Sonde 14 ist bereits eine erste Fixierung der Sonde 14 im Körper des Lebewesens gewährleistet; nämlich zumindest eine Fixierung innerhalb einer von zwei unabhängigen Koordinaten aufgespannten Ebene. Mit anderen Worten sind die Bewegungsmöglichkeiten der Sonde 14 bereits durch die Rinnenform des Fortsatzes 4 und der Anpassung der Fortsatzanlagefläche 4" an die Sonde 14 auf diejenige Ebene beschränkt, die durch die beiden folgenden unabhängigen Koordinaten aufgespannt wird, "Rinnenlängsachse" und "Winkelhalbierende des den Rinnenumfang beschränkenden Zentriwinkels". Da der Rinnenboden diese Ebene nach unten begrenzt, kann sich die Sonde 14 innerhalb dieser Ebene nur noch vom Rinnenboden weg nach oben bewegen. Eine zusätzliche Fixierung in Richtung einer dritten unabhängigen Koordinate, d.h. eine vollständige Fixierung im Raum, wird durch zwei Streifen aus Heftpflaster 19 erreicht. Dabei können die einen Enden der beiden Streifen von vornherein fest auf dem Flächengebilde 1 verankert sein, während der Rest der Heftpflasterstreifen 19 vor Inbetriebnahme des erfindungsgemäßen Verbandes mit einem Silikonpapier beschichtet ist. Nach Entfernen des Silikonpapiers von den Heftpflasterstreifen 19 können diese über die Kanüle 14 geführt und auf der - der den von vornherein festgelegten Enden der Heftpflasterstreifen 19 bezüglich des Fortsatzes 4 - gegenüberliegenden Seite des Plättchens befestigt werden.

Das dargestellte Ausführungsbeispiel stellt eine ebenso einfache wie sichere Festlegung einer in die Haut 20 eines Lebewesens eingeführten Sonde sicher. Eine derartige Festlegung dient der Schonung des mit der Sonde versehenen Lebenswesens unter mehreren Gesichtspunkten. Zunächst einmal wird eine Schmerzen verursachende Relativbewegung zwischen Sonde und Haut weitgehend vermieden. Hinzu kommt eine Schonung der Vene durch Verminderung bewegungsbedingter Reizungen der Vene sowie insbesondere Reduzierung der Gefahr eines Venendurchstiches - mit der Folge der Verminderung der Gefahr einer lebensbedrohlichen Trombosenbildung. Durch die Ruhigstellung der in den Körper eingeführten Sonde wird gleichzeitig die Verschlußbildung zwischen der Haut und dem Sondenkörper an der Einstichstelle verbessert und damit die Gefahr eines Eindringens pathogener Fremdkörper an dieser Stelle. Auch dieser Vorteil dient letztlich der Schonung des jeweils mit der Sonde versehenen Patienten. Infolge der Fixierung der Sonde in allen drei Raumkoordinaten wird auch ein Abknicken der Sonde und damit eine Unterbrechung einer durch die Sonde in den Körper des Lebewesens eingeführten Substanz oder - in umgekehrter Richtung - eines Staus einer aus dem Körper des Lebewesens ausgeführten Substanz beseitigt. Entsprechendes gilt für den Fall, daß die Sonde weder der Zuführung noch der Abführung von Substanzen in oder aus dem Körper dient, sondern der Ab- und Weiterleitung von Signalen, die Auskunft über Funktionsabläufe im Körper geben.

Darüberhinaus läßt sich der erfindungsgemäße Verband schnell, einfach und sicher anlegen, nämlich dadurch, daß er nach Einführung der Sonde in den Körper unter den aus der Haut vorstehenden Teil der Sonde geschoben und dann in der beschriebenen Weise befestigt wird. Auch dieses rasche Befestigen des Verbandes mit der Sonde dient der Schonung des Patienten.

Gemäß einem weiteren bevorzugten Ausführungsbeispiel ist das Plättchen bzw. das Flächengebilde 1 auf seiner Unterseite frei von einer Haftschicht 10. In diesem Fall wird es - in gleicher Weise wie die Sonde auf dem Fortsatz 4 mittels der Heftpflasterstreifen 19 - mit Haftstreifen auf der Haut 20 befestigt.

Institut für wissenschaftliche
Photographie und Kinematographie
Willi Fischer

0121679

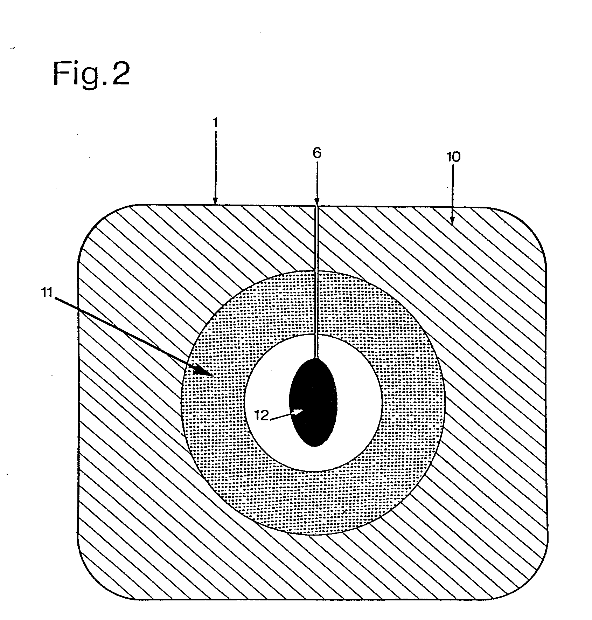

Gemäß Figur 2 weist die Folie, d.h. das Flächengebilde 1 eine mittig angeordnete ovale Durchtrittsöffnung 12 für den Durchtritt der Sonde, insbesondere also der Kanüle 14, auf. Infolge eines in Richtung der großen Längsachse der ovalen Durchtrittsöffnung 12 von der Außenkante des Flächengebildes 1 bis hin zum äußeren Rand der Durchtrittsöffnung 12 geführten Einschnittes ist die Folie mit wenigstens einer längs der Schnittlinie laufenden freien Innenkante 6 versehen. Die dieser freien Innenkante 6 gegenüberliegende Hälfte der Folie ist mit einer die freie Innenkante 6 überlappenden Lasche 2 bestückt. Durch Anheben der Lasche 2 entsteht ein zur freien Innenkante 6 der Folie im wesentlichen parallel verlaufender und bis zur ovalen Durchtrittsöffnung 12 führender Schlitz, der ein späteres Unterschieben des Verbandes unter den aus der Haut 20 herausragenden Teil der Sonde erleichtert. Zur späteren Befestigung der Lasche 2 an der Folie ist die Lasche auf ihrer Unterseite mit einer - vorzugsweise streifenförmigen - Haftschicht 7 ausgestattet. Die Lasche 2 hat hierbei im wesentlichen den Zweck einer vollständigen Abdeckung des Applikationsortes sowie einer Erleichterung der Ablösung der Folie von der Haut 20. Dem letztgenannten Zweck dient auch die streifenförmige Ausbildung der Haftschicht 7.

Ein gegenüber den in den Figuren abgewandeltes Ausführungsbeispiel des erfindungsgemäßen Verbandes weist keine Lasche 2 auf. In diesem Fall wird die von der freien Kante 6 der Folie bis hin zum äußeren Rand der Durchtrittsöffnung 12 führende Schnittnaht von zwei freien Innenkanten 6 begrenzt (siehe auch Figur 2).

Eine vollständige Abdeckung des Applikationsortes ist hierbei dadurch erreichbar, daß die beiden freien Innenkanten des Flächengebildes 1 in Wirkstellung

0121679

bündig aneinander grenzen.

Gemäß Figur 2 weist das dargestellte Ausführungsbeispiel des erfindungsgemäßen Verbandes auf seiner Unterseite wiederum eine hautfreundliche Haftschicht 10 auf. Zusätzlich ist die Durchtrittsöffnung 12 von einem ringförmigen Vlies 11 umgeben, das bei entsprechendem Aneinanderfügen der beiden freien Innenkanten 6 bzw. der einen freien Innenkante 6 und des restlichen Teiles des Flächengebildes 1 lückenlos aneinanderschließt. Da das Vlies 11 selber zwei freie Kanten aufweist, wird durch die hierdurch vergrößerte Fläche der freien Kanten ein lückenloses Aneinanderfügen besonders erleichtert. Das Vlies 11 ist mit entzündungshemmenden oder antibiotischen oder einer Mischimprägnierung aus Antibiotika und entzündungshemmenden Pharmaka präpariert. Das die Punktionsstelle ringförmig umgebende Vlies 11 dient der zusätzlichen Abschirmung der Punktionsstelle gegen pathogene Erreger.

Sämtliche Haftschichten sind, soweit sie für direkten Kontakt mit der Haut 20 vorgesehen sind, als hautfreundliche Haftschichten ausgebildet. Im übrigen sind die Haftschichten mit einem sterilen Silikonpapier abgedeckt, das nicht nur eine Kontamination der Haftschichten mit pathogenen Substanzen, sondern auch ein Zusammenkleben der Haftschichten bei der Konfektionierung verhindert.

Das Silikonpapier wird in üblicher Weise erst unmittelbar vor dem Anbringen der Haftschichten auf der Haut entfernt.

Die in den Figuren dargestellten Ausführungsbeispiele des erfindungsgemäßen Verbandes weisen eine dünne elastische Folie als Flächengebilde 1 auf. Je nach Bedarf ist die Folie dem Applikationsort angepaßt, d.h. sie kann rund, oval, dreieckig oder viereckig geschnitten sein. Die Folie überdeckt hierbei den gesamten Applikationsort. Um auch nach Anbringung des Verbandes den gesamten Applikationsort gut beobachten, insbesondere unerwünschte Veränderungen wie Entzündungen, Trombosebildungen oder dergleichen bequem beobachten zu können, ist die Folie durchsichtig, insbesondere auch röntgenstrahlendurchlässig. Die Röntgenstrahlendurchlässigkeit ermöglicht darüberhinaus auch eine transvenöse Extraktion der Tromben usw. durch Schlingen. Bevorzugt besteht die Folie aus Silikon-Kautschuk, Latex-Kautschuk, Polyurethan, Polytetrafluoräthylen oder ähnlichen Werkstoffen. Nur am Rande sei erwähnt, daß der für die Folie verwendete Werkstoff zur Vermeidung von Intoxikationen keine Weichmacher enthält.

C121679
08.02.1984

P a t e n t a n s p r ü c h e

1.    Medizinischer Verband zur Fixierung einer durch die Haut (20) eines Lebewesens in dessen Körper eingeführten Sonde (14) mit

a) einem an der Haut (20) und am aus der Haut (20) herausragenden Teil der Sonde (14) gleichzeitig befestigbaren Verbandsabschnitt (1, 4),

b) der ein zur Anlage an der Haut (20) ausgelegtes Flächengebilde (1) und

c) einen mit dem Flächengebilde (1) verbundenen, von diesem stutzenartig vorstehenden Fortsatz (4) mit

c.1) einer daran zur Anlage an den aus der Haut (20) herausragenden Teil der Sonde ausgeformten Anlagefläche (4")

aufweist,

dadurch gekennzeichnet, daß

d) der Fortsatz (4) nach Art einer oben offenen, den aus der Haut (20) herausragenden Teil der Sonde (14) abstützenden Rinne ausgebildet ist.

2.    Medizinischer Verband nach Anspruch 1, dadurch gekennzeichnet, dass der Fortsatz (4) über eine Verbindungsrippe (18) mit dme Flächengebilde (1) verbunden ist.

3.    Medizinischer Verband nach Anspruch 1 oder 2, dadurch gekennzeichnet, dass die Anlagefläche (4") eine Haftschicht aufweist.

4.    Medizinischer Verband nach einem der vorstehenden Ansprüche, dadurch gekennzeichnet, dass der Fussbereich des Fortsatzes (4) allseitig vom Flächengebilde (1) umgeben ist und das Flächengebilde (1) in diesem Bereich eine Durchtrittsöffnung (12) aufweist.

0121679

5.    Medizinischer Verband nach einem der vorstehenden Ansprüche, dadurch gekennzeichnet, dass der Fortsatz (4) aus demselben Material wie das Flächengebilde (1) besteht.

Fig.1

Fig.2

## EINSCHLÄGIGE DOKUMENTE

EP 84101298.2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y,D | US - A - 3 683 911 (J. CORMICK)  <br> * Gesamt * <br> -- | 1,3-5 | A 61 M 25/02 <br> A 61 F 13/00 <br> A 61 F 13/02 |
| Y | DE - B - 1 954 956 (DOW CORNING) <br> * Fig. 5,6,10; Spalte 4, Zeilen 4-25 * | 1,3-5 | |
| D | & US-A-3 568 679 <br> -- | | |
| A | US - A - 4 261 363 (R. RUSSO) <br> * Gesamt *. <br> -- | 1,2,5 | |
| A | US - A - 3 702 612 (R. SCHLESINGER) <br> * Gesamt * <br> -- | 1,2 | |
| A,D | US - A - 3 834 380 (W. BOYD) <br> * Gesamt * <br> -- | 1 | **RECHERCHIERTE SACHGEBIETE (Int. Cl. ³)** <br> A 61 M 25/00 <br> A 61 F 13/00 <br> A 61 M 5/00 |
| A | US - A - 4 250 880 (M. GORDON) <br> * Fig. 11-15; Spalte 4, Zeilen 21-47 * <br> -- | 1,5 | |
| A | US - A - 3 630 195 (L. SANTOMIERI) <br> * Gesamt * <br> -- | 1 | |
| A | GB - A - 904 237 (CH. ELLIOT) <br> * Fig. 1; Anspruch 1 * <br> ---- | | |

Der vorliegende Recherchenbericht wurde für alle Patentansprüche erstellt.

| Recherchenort | Abschlußdatum der Recherche | Prüfer |
|---|---|---|
| WIEN | 23-07-1984 | LUDWIG |